# DEMANDE DE BREVET EUROPEEN

(11) **EP 2 287 336 A1**
(43) Date de publication de la demande: **23.02.2011**
(21) Numéro de dépôt: 09305721.4
(22) Date de dépôt: 31.07.2009
(51) Int. Cl.: C12Q 1/68

(54) **Procédés et méthodes de diagnostic de la maladie d'Alzheimer**

(71) Demandeur: Exhonit Therapeutics SA, 75017 Paris (FR)
(72) Inventeur: Einstein, M. Rich, Gaithersburg, MD 20878 (US); Zhou, Weiyin, Derwood, MD 20855 (US); Beurdeley, Pascale, 94120 Fontenay sous Bois (FR)
(74) Mandataire: Becker, Philippe

(57) **Abrégé**

La présente demande concerne des méthodes et compositions utilisables pour le diagnostic de la maladie d'Alzheimer chez les mammifères, en particulier les humains. Elle décrit notamment des marqueurs sériques de la maladie d'Alzheimer et leurs utilisations dans des méthodes de diagnostic. Elle concerne également des outils et/ou kits utilisables pour la mise en oeuvre de ces méthodes (réactifs, sondes, amorces, anticorps, puces, cellules, etc.), leur préparation et leurs utilisations. L'invention est utilisable pour détecter la présence ou l'évolution de la maladie d'Alzheimer chez les mammifères, y compris en phase précoce, ainsi que pour la prédiction d'efficacité de traitement de la maladie d'Alzheimer.

## Description

La présente demande concerne des méthodes et compositions utilisables pour la détection de la maladie d'Alzheimer chez les mammifères, en particulier les humains. Elle décrit notamment des marqueurs sériques de la maladie d'Alzheimer et leurs utilisations dans des méthodes de diagnostic. Elle concerne également des outils et/ou kits utilisables pour la mise en oeuvre de ces méthodes (réactifs, sondes, amorces, anticorps, puces, cellules, etc.), leur préparation et leurs utilisations. L'invention est utilisable pour détecter la présence ou l'évolution de la maladie d'Alzheimer chez les mammifères, y compris en phase précoce.

La maladie d'Alzheimer représente la principale cause de démence et la maladie neurodégénérative la plus fréquente. Cette maladie, d'évolution progressive, est caractérisée par la perte de mémoire et par une dégradation des aptitudes au langage, à l'orientation et au jugement. La nature des symptômes, souvent confondus avec les désagréments physiologiques liés à la vieillesse, leur sévérité ainsi que l'âge de leurs apparitions, varient selon les individus. Ceci contribue à la difficulté d'établir un diagnostic aux stades précoces de la maladie.

L'examen de cerveaux de patients atteints de cette maladie révèle une perte de neurones de l'hippocampe, centre important de la mémoire, et du cortex cérébral, impliqué dans le raisonnement, le langage et la mémoire. Les neurones cholinergiques sont particulièrement affectés par cette déplétion.

Une autre anomalie majeure observée dans les cerveaux des malades atteints de la maladie d'Alzheimer est l'accumulation d'agrégats intracellulaires et extracellulaires de protéines. Des agrégats neurofibrillaires intracellulaires de la protéine tau semblent bien corrélés avec la gravité de la démence. Des plaques séniles formées par agrégation intra et extracellulaire de peptide beta-amyloïde caractérisent des régions d'altérations de neurones et de cellules gliales.

Il est cependant remarquable de noter que ces zones d'agrégation ne correspondent pas aux sites de la déplétion en synapses caractéristique du déclin des fonctions cognitives.

Les études génétiques menées sur les formes familiales ont montré que 4 gènes sont associés au développement de la maladie. L'APP (Amyloid Precursor Protein ; précurseur du peptide beta amyloide), les présénilines 1 et 2 (PSI et PS2) et l'apolipoprotéine E (ApoE). Bien que des mutations ou polymorphismes dans chacun de ces gènes aboutissent à une production accrue de peptide beta amyloïde, les mécanismes qui président aux pertes synaptiques et neuronales restent mal connus. A cet égard, plusieurs hypothèses et mécanismes semblent ainsi coexister, qui impliquent différents phénomènes:
1 Des phénomènes purement cérébraux impliquant les neurones et les cellules gliales:
   - le stress oxydatif, qui peut être induit notamment par le peptide beta amyloïde et modulé par le métabolisme du cholestérol;
   - des modifications de flux calciques et l'excitotoxicité.
2 Des phénomènes inflammatoires et immunitaires réactionnels ;
3 Une altération des hormones sexuelles;
4 L'hypothyroïdisme et des défauts dans la régulation des signaux insuliniques.

Par conséquent, la maladie d'Alzheimer serait caractérisée par une altération de différents systèmes d'intégration régulant l'homéostasie, l'atteinte de certains neurones entraînant à la fois une réaction inflammatoire impliquant le système immunitaire et des modifications des régulations endocriniennes. Ces dernières ont en retour un impact sur l'activité et la viabilité d'autres neurones et sur les fonctions immunitaires, ces réactions en cascade soulignant le rôle non seulement de la neurodégénérescence mais aussi des régulations hormonales et de la réponse immunitaire dans la progression de la maladie d'Alzheimer.

Il n'existe actuellement aucune signature robuste et spécifique de la maladie d'Alzheimer, notamment à partir d'échantillon sanguin, susceptible de permettre d'établir un diagnostic de cette pathologie, notamment des différents stades de l'évolution de la maladie. La mise à disposition d'un test de diagnostic efficace, notamment précoce, permettrait aux patients d'être pris en charge dès le début de la maladie, et de bénéficier ainsi d'un traitement plus efficace et plus adapté, notamment par des inhibiteurs d'acétylcholinestérase tels que la galantamine, le donepezil et la rivastigmine, dans des conditions optimales.

La présente invention apporte une réponse à ce besoin. L'invention décrit notamment l'identification de marqueurs sériques de la maladie d'Alzheimer, permettant la mise au point de diagnostics efficaces et prédictifs de la présence, du degré de sévérité / avancement ou du risque de développer cette maladie. L'invention décrit ainsi l'identification de signatures moléculaires spécifiquement ou préférentiellement exprimées dans le sang de patients atteints de la maladie d'Alzheimer, résultant notamment de l'expression complexe de certains gènes liée à des épissages alternatifs. L'invention décrit notamment l'identification des séquences SEQ ID Nos : 1-1754, qui sont présentes dans des gènes ou des ARN de cellules sanguines de sujets humains, et qui sont caractéristiques, seules ou en combinaisons, de la maladie d'Alzheimer. L'invention peut donc proposer des outils et méthodes de diagnostic, de prédiction et/ou de suivi de l'évolution de la maladie d'Alzheimer, basés sur une mesure, dans le sang de sujets, de l'expression d'un ou plusieurs gènes. La présence d'une dérégulation dans l'expression de tels gènes permet d'établir (ou de confirmer) la présence de la maladie d'Alzheimer chez un sujet.

Un objet de l'invention réside ainsi dans une méthode pour détecter ou confirmer (*in vitro* ou *ex vivo*) la présence de la maladie d'Alzheimer chez un mammifère, comprenant la détermination de la présence, dans un échantillon biologique du mammifère, de préférence dans un échantillon (dérivé) de sang, d'une altération dans un ou plusieurs gènes ou ARN comprenant une séquence choisie parmi SEQ ID Nos : 1-1754, la présence d'une telle altération étant indicative de la présence ou du risque de développer la maladie d'Alzheimer chez ce mammifère.

Un autre objet de l'invention concerne une méthode pour évaluer ou suivre la réponse à un traitement de la maladie d'Alzheimer, comprenant une étape de mesure de l'expression d'un ou, de préférence, de plusieurs gènes ou ARN comprenant une séquence choisie parmi SEQ ID Nos : 1 à 1754 avant et/ou au cours du traitement, et une comparaison de l'expression ainsi mesurée à celle mesurée à un stade antérieur du traitement ou au traitement, une modification de ladite expression étant indicative d'une réponse au traitement.

Un autre objet de l'invention concerne une amélioration aux méthodes de traitement de la maladie d'Alzheimer, l'amélioration consistant à mesurer l'expression d'un ou, de préférence, de plusieurs gènes ou ARN comprenant une séquence choisie parmi SEQ ID Nos : 1 à 1754, chez un sujet, avant et/ou pendant le traitement. La mesure de l'expression permet d'adapter le traitement en fonction de l'évolution de la pathologie. Le traitement est typiquement un traitement par des inhibiteurs d'acétylcholinestérase, tels que la galantamine, le donepezil et la rivastigmine.

Un autre objet de l'invention concerne l'utilisation d'un inhibiteur d'acétylcholinestérase, tel que la galantamine, le donepezil et la rivastigmine, pour la préparation d'un médicament pour traiter la maladie d'Alzheimer chez un patient présentant une dérégulation de l'expression d'un gène (au moins) tel que défini précédemment.

L'altération dans un gène ou ARN désigne au sens de l'invention (i) toute altération de l'expression, à savoir en particulier une dérégulation dans les niveaux d'expression (e.g., de transcription ou de traduction), une dérégulation de l'épissage, conduisant par exemple à l'apparition de formes épissées particulières ou à une modification de la quantité (relative) ou du rapport entre les différentes formes d'épissage, de même que (ii) toute altération dans la structure de la protéine produite (apparition ou disparition de formes tronquées, allongées, mutées, etc.).

Comme il sera décrit dans la suite du texte, la présente demande décrit l'identification de dérégulations de l'épissage parmi certains gènes dans le sang de patients atteints de la maladie d'Alzheimer. Toute molécule ou technique permettant de mesurer l'expression de ces gènes dans le sang peut être mise en oeuvre dans le cadre de la présente invention, telles que des amorces nucléotidiques, des sondes nucléotidiques ou des anticorps spécifiques, qui peuvent être en suspension ou sous forme immobilisée, comme il sera décrit en détails dans la suite du texte.

Ainsi, un autre objet de la présente demande concerne un produit comprenant un support sur lequel sont immobilisés des acides nucléiques comprenant une séquence complémentaire et/ou spécifique d'un ou, de préférence, plusieurs gènes ou ARN tels que définis précédemment. De préférence, le produit comprend des acides nucléiques distincts comprenant une séquence complémentaire et/ou spécifique d'au moins 5, 10, 20, 30, 40, 50, 60, 100, 150, 200 ou plus gènes ou ARN tels que définis précédemment.

Un autre objet de la présente demande concerne un produit comprenant un support sur lequel est immobilisé au moins un ligand d'un polypeptide codé par un gène ou un ARN tel que défini ci-dessus. De préférence, le produit comprend au moins 5, 10, 20, 30, 40, 50, 60 ou plus ligands de polypeptides différents choisis parmi les polypeptides mentionnés ci-dessus.

Un autre objet de la présente demande concerne un kit comprenant un compartiment ou conteneur comprenant au moins un, de préférence plusieurs, acides nucléiques comprenant une séquence complémentaire et/ou spécifique d'un ou plusieurs gènes ou ARNs tels que définis précédemment et/ou un, de préférence plusieurs ligands d'un ou plusieurs polypeptides tels que définis précédemment. De préférence, le produit comprend au moins 5, 10, 20, 30, 40, 50, 60, 100, 150, 200 ou plus de séquences d'acides nucléiques et/ou ligands différents choisis parmi les acides nucléiques et ligands mentionnés ci-dessus. Le kit peut comprendre par ailleurs des réactifs pour une réaction d'hybridation ou immunologique, ainsi que, le cas échéant, des contrôles et/ou instructions.

Un autre objet de l'invention concerne l'utilisation d'un produit ou kit tel que défini ci-dessus pour la détection de la maladie d'Alzheimer chez un sujet mammifère, de préférence un sujet humain.

Un autre objet de l'invention concerne l'utilisation d'un produit ou kit tel que défini ci-dessus pour la détermination de la réponse à un traitement de la maladie d'Alzheimer ou pour la sélection de sujets susceptibles de répondre de façon efficace à un traitement.

Un autre objet de l'invention réside dans un acide nucléique isolé comprenant une séquence choisie parmi SEQ ID Nos : 1 à 1754 ou un fragment de celle-ci ayant au moins 15, 16, 17, 18, 19 ou 20 bases consécutives, ou une séquence complémentaire de celles-ci. De préférence, l'acide nucléique de l'invention ne comprend pas la séquence complète d'un gène ou ARN naturel. De préférence, il ne comprend pas plus de 500 bases, de préférence pas plus de 400 ou 300 bases. L'acide nucléique de l'invention est typiquement synthétique, c'est-à-dire produit par voie non naturelle (recombinante, in vitro, synthèse chimique, etc.).

Un autre objet de l'invention réside dans un polypeptide codé par un acide nucléique tel que défini ci-dessus.

### Marqueurs sériques de la maladie d'Alzheimer

La présente invention repose sur la mise en évidence et la caractérisation d'événements biologiques sériques caractéristiques de la maladie d'Alzheimer chez un patient humain. Ces événements constituent des biomarqueurs, dont la détection chez un patient permet, de préférence en combinaison, de déterminer, même à un stade précoce, la présence d'une telle maladie, ou le stade d'évolution de cette maladie. En outre, les marqueurs selon l'invention sont également utilisables pour mesurer la réponse à un traitement, et/ou pour sélectionner des médicaments candidats.

Les événements biologiques identifiés correspondent typiquement à des modifications dans la régulation de l'expression de gènes. Il peut s'agir d'une inhibition partielle ou totale de l'expression de gènes ou d'ARN, ou de certaines formes de gènes ou d'ARN, d'une augmentation de l'expression de gènes ou de certaines formes de gènes ou d'ARN, de l'apparition ou de la disparition de formes d'épissages de gènes, etc.

L'invention repose donc sur la détection, dans un échantillon, d'une ou plusieurs molécules cibles choisies avantageusement parmi :
a) les acides nucléiques comprenant une séquence choisie parmi SEQ ID NOs: 1 à 1754, ou un fragment distinctif de ceux-ci ayant au moins 15, de préférence au moins 16, 17, 18, 19, 20, 25 ou 30 bases consécutives,
b) les acides nucléiques ayant une séquence complémentaire d'une séquence selon a),
c) les analogues fonctionnels d'acides nucléiques selon a) ou b), ou
d) les polypeptides codés par les acides nucléiques selon a) à c).

Les acides nucléiques de séquences SEQ ID Nos : 1-1754 ont été identifiés par les inventeurs à partir d'échantillons de cellules sanguines humaines saines ou de sujets atteints de la maladie d'Alzheimer, par des techniques d'analyse du génome et du transcriptome. Ces séquences sont caractéristiques, en combinaisons, de la maladie d'Alzheimer. Ces séquences sont désignées dans la présente demande par séquences « cibles » (ou « target »). Elles sont présentées dans le sens biologique du gène. Leur séquence est donnée dans le séquence listing, et leur description dans le tableau 1.

Le terme « analogue fonctionnel » désigne de préférence un variant polymorphique des séquences SEQ ID NO : 1 à 1754 présent parmi la population humaine. Dans la plupart des cas, ces polymorphismes sont représentés par des variations ponctuelles au niveau d'une base, même si d'autres configurations polymorphiques existent également. Ces analogues peuvent être identifiés par toute technique connue de l'homme du métier, notamment en considération des séquences fournies dans la demande et des noms des gènes correspondants.

Dans un mode de réalisation particulier, la méthode comprend la détermination de la présence (ou de l'absence ou d'une variation de niveau d'expression) d'au moins un acide nucléique selon a) à c).

Dans un mode de réalisation tout particulier, la méthode est utilisée pour détecter la maladie d'Alzheimer chez un sujet humain et comprend la détermination de la présence (ou de l'absence ou d'une variation de niveau d'expression) d'au moins un acide nucléique selon a) à c).

Dans une variante particulière de mise en oeuvre, la méthode comprend la détermination combinée de la présence ou absence ou quantité (relative) d'au moins 5, 10, 15, 20, 30, 40, 50, 60, 70 ou plus des molécules cibles telles que définies ci-dessus. La détermination "combinée" désigne le fait qu'un profil d'hybridation (ou une signature) impliquant plusieurs marqueurs est déterminé. La détermination combinée est typiquement réalisée de manière simultanée, c'est-à-dire par mesure globale d'un profil d'expression. Néanmoins, la détermination combinée peut également être effectuée par mesures en parallèle ou séquentielle de plusieurs marqueurs, conduisant à l'identification d'un profil. L'invention permet en effet d'établir et de déterminer un profil d'hybridation (ou une signature) sur un ensemble de marqueurs, afin d'évaluer la présence ou le risque de développer la maladie d'Alzheimer chez un mammifère. Le profil d'hybridation est typiquement réalisé en utilisant une combinaison de plusieurs marqueurs choisis parmi les cibles indiquées ci-dessus, par exemple contenant l'ensemble de ces cibles.

Dans un mode de mise en oeuvre particulier, la méthode de l'invention comprend la détermination de la présence (ou de l'absence ou de la quantité (relative)), dans un échantillon biologique du mammifère, d'au moins 5 molécules cibles distinctes choisies parmi celles définies ci-dessus, de préférence d'au moins 10.

A cet égard, la présente demande décrit des sous-ensembles (« panels ») particuliers de molécules cibles choisies parmi celles définies ci-dessus, qui sont particulièrement adaptés à la détection de la présence de la maladie d'Alzheimer chez des patients à partir d'un échantillon de sang total.

Ainsi, dans un mode de mise en oeuvre particulier, la méthode de l'invention comprend la détermination combinée de la présence ou de l'absence ou de la quantité relative, dans un échantillon biologique du mammifère, des acides nucléiques de l'ensemble d'un panel de cibles comprenant des marqueurs tels que définis aux éléments a) à d) ci-dessus, de préférence de l'ensemble des acides nucléiques de l'un des panels 1 à 16 définis dans la présente demande (voir tableau 2).

Ainsi, dans un mode particulier, la méthode de l'invention comprend la détermination combinée de la présence (ou de l'absence ou de la quantité (relative)), dans un échantillon biologique du mammifère, de l'ensemble des acides nucléiques d'un des Panels 1 à 14, ou un fragment distinctif de celles-ci ayant au moins 15, de préférence au moins 16, 17, 18, 19, 20, 25 ou 30 bases consécutives, ou ayant une séquence complémentaire de celles-ci et/ou des analogues fonctionnels de ceux-ci, et/ou des polypeptides codés par ces acides nucléiques. Les exemples fournis dans la présente demande montrent en effet que ces panels de marqueurs permettent de détecter de manière prédictive la présence ou le stade d'évolution de la maladie d'Alzheimer. Dans un mode particulier, la méthode comprend en outre la détection d'une ou plusieurs des autres molécules cibles telles que définies précédemment.

Dans un mode spécifique de mise en oeuvre, la méthode de l'invention comprend la détermination de la présence (ou de l'absence ou de la quantité (relative)), dans un échantillon biologique du mammifère, des acides nucléiques comprenant respectivement les séquences représentées dans SEQ ID NOs: 1 à 1754 ou un fragment distinctif de celles-ci ayant au moins 15, de préférence au moins 16, 17, 18, 19, 20, 25 ou 30 bases consécutives, ou des acides nucléiques ayant une séquence complémentaire de celles-ci.

Un objet particulier de l'invention réside dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins une sonde comprenant tout ou partie de chacune des séquences nucléiques cibles suivantes, ou de leur brin complémentaire : SEQ ID NOs : 34, 230, 341, 454, 664, 811, 951, 1127, 1136 et 1752,
pour obtenir un profil d'hybridation, le profil d'hybridation étant caractéristique de la présence de la maladie d'Alzheimer chez ce mammifère.

Dans un mode de mise en oeuvre préféré de la méthode, l'ensemble de sondes comprend, en plus des sondes indiquées ci-dessus, au moins une sonde comprenant tout ou partie de chacune des séquences nucléiques cibles supplémentaires suivantes, ou de leur brin complémentaire : SEQ ID Nos : 35 ; 316 ; 593 ; 666 ; 855 ; 1330 et 1498.

Ainsi, un panel préféré d'acides nucléiques cibles comprend au moins les acides nucléiques comprenant les séquences SEQ ID NOs : 34, 35 ; 230, 316 ; 341, 454, 593 ; 664, 666 ; 811, 855 ; 951, 1127, 1136 ; 1330 ; 1498 et 1752, ou leur séquence complémentaire. Comme illustré dans les exemples, différents panels de marqueurs ont été mis en évidence et validés cliniquement par les inventeurs, permettant une détermination de la présence de la maladie d'Alzheimer chez un sujet. Les panels 15 et 16 définis ci-dessus comprennent un ensemble de marqueurs communs à tous les panels significatifs 1-14 décrits et testés cliniquement dans la présente demande, et constituent ainsi deux sous-ensembles particulièrement informatifs et représentatifs pour la détection de la maladie d'Alzheimer.

De manière particulièrement préférée, l'ensemble de sondes comprend au moins une sonde spécifique de chaque acide nucléique de l'un des panels 1 à 14 définis dans le tableau 2, ou d'un fragment distinctif de ceux-ci ayant au moins 15, de préférence au moins 16, 17, 18, 19, 20, 25 ou 30 bases consécutives, et/ou de leur brin complémentaire.

Par ailleurs, comme il sera expliqué plus en détails dans la suite du texte, on utilise avantageusement des groupes de sondes (« probe sets ») comprenant, pour chaque séquence cible d'acides nucléiques, plusieurs sondes spécifiques partiellement chevauchantes ou non chevauchantes, typiquement de 1 à 3. Par ailleurs, les sondes sont avantageusement immobilisées sur un support. En outre, le profil d'hybrisation est généralement analysé par des méthodes informatiques.

Un autre objet de l'invention réside dans une méthode pour détecter la présence ou le risque de développer la maladie d'Alzheimer chez un mammifère, la méthode comprenant la détection, à partir d'un échantillon de sang du mammifère, d'une variation dans les niveaux d'acides nucléiques complémentaires d'un groupe de sondes tel que défini précédemment, une variation étant caractéristique de la présence de la maladie d'Alzheimer chez ce mammifère.

L'invention permet également la définition de panels supplémentaires, comprenant au moins certains marqueurs tels que définis précédemment, qui peuvent éventuellement être combinés à d'autres marqueurs. De tels panels peuvent être obtenus en testant la présence ou non de ces marqueurs dans des échantillons de patients, pour définir d'autres combinaisons prédictives, le cas échéant spécifiques de pathologies particulières.

L'invention concerne encore l'utilisation d'un ensemble de sondes tel que défini précédemment pour détecter *in vitro* ou *ex vivo* la présence de la maladie d'Alzheimer chez un sujet.

L'invention concerne en outre l'utilisation d'un ensemble d'amorces comprenant au moins une amorce comprenant tout ou partie d'une ou plusieurs des séquences nucléiques cibles mentionnées précédemment, pour la détection *in vitro* ou *ex vivo* de la maladie d'Alzheimer.

L'invention concerne également une méthode pour détecter la présence de la maladie d'Alzheimer chez un mammifère, comprenant la mise en contact, dans des conditions permettant une réaction d'amplification, des acides nucléiques issus d'un échantillon de sang du mammifère et d'un ensemble d'amorces, l'ensemble d'amorces comprenant au moins une amorce comprenant tout ou partie de chacune des séquences nucléiques cibles suivantes, ou de leur brin complémentaire : SEQ ID NOs : 34, 230, 341, 454, 664, 811, 951, 1127, 1136 et 1752,
pour obtenir un profil ou produit d'amplification, le profil ou produit d'amplification étant caractéristique de la présence de la maladie d'Alzheimer chez ce mammifère.

### Méthodes de détection d'une altération dans un gène

Comme indiqué précédemment, une altération dans un gène ou ARN désigne au sens de l'invention (i) toute altération de l'expression, à savoir en particulier une dérégulation dans les niveaux d'expression (e.g., de transcription ou de traduction), une dérégulation de l'épissage, conduisant par exemple à l'apparition de formes épissées particulières ou à une modification de la quantité (relative) de ou du rapport entre différentes formes d'épissage, de même que (ii) toute altération dans la structure de la protéine produite (apparition ou disparition de formes tronquées, allongées, mutées, etc.).

Différentes techniques permettant la détection d'une espèce d'acide nucléique dans un échantillon sont utilisables dans la présente invention, comme par exemple le Northern Blot, l'hybridation sélective, l'utilisation de supports revêtus d'oligonucléotides sondes, l'amplification d'acide nucléique comme par exemple par RT-PCR, PCR quantitative ou ligation-PCR, etc. Ces méthodes peuvent comprendre l'utilisation d'une sonde nucléique (par exemple un oligonucléotide) capable de détecter sélectivement ou spécifiquement l'acide nucléique cible dans l'échantillon. L'amplification peut être réalisée selon différentes méthodes connues en soi de l'homme du métier, telles que la PCR, la LCR, l'amplification médiée par transcription (TMA), l'amplification par déplacement de brin (SDA), NASBA, l'emploi d'oligonucléotides spécifiques d'allèles (ASO), l'amplification spécifique d'allèle, le Southem blot, l'analyse conformationnelle SSCA, l'hybridation in situ (e.g., FISH), la migration sur gel, l'analyse d'hétéroduplexes, etc. Si nécessaire, la quantité d'acide nucléique détectée peut être comparée à une valeur de référence, par exemple une valeur médiane ou moyenne observée chez des patients qui ne sont pas atteints de la maladie d'Alzheimer, ou à une valeur mesurée en parallèle dans un échantillon témoin. Ainsi, il est possible de mettre en évidence une variation de niveaux d'expression.

Selon un mode préféré de mise en oeuvre, la méthode comprend la détection de la présence ou de l'absence ou de la quantité (relative) d'un acide nucléique selon a) à c) par hybridation sélective ou amplification sélective.

L'hybridation sélective est typiquement réalisée en utilisant des sondes nucléiques, de préférence immobilisées sur un support, tel qu'un support solide ou semi-solide présentant au moins une surface, plane ou non, permettant l'immobilisation de sondes nucléiques. De tels supports sont par exemple une lame, bille, membrane, filtre, colonne, plaque, etc. Ils peuvent être réalisés en tout matériau compatible, comme notamment du verre, silice, plastique, fibre, métal, polymère, etc. Les sondes nucléiques peuvent être tout acide nucléique (ADN, ARN, PNA, etc.), de préférence simple-brin, comprenant une séquence spécifique d'une molécule cible telle que définie en a) à c) ci-dessus. Les sondes comprennent typiquement de 5 à 400 bases, de préférence de 8 à 200, plus préférentiellement moins de 100, et encore plus préférentiellement moins de 75, 60, 50, 40 ou même 30 bases. Les sondes peuvent être des oligonucléotides synthétiques, produits sur la base des séquences SEQ ID NO : 1 à 1754 (target sequences) de l'invention, selon des techniques de synthèse classique. De tels oligonucléotides comportent typiquement de 10 à 50 bases, de préférence de 20 à 40, par exemple 25 bases environ. Dans un mode particulièrement avantageux, de manière à améliorer le signal détecté, on utilise plusieurs oligonucléotides (ou sondes) différents définis à partir d'une même séquence cible (target sequence) pour détecter la même molécule cible (transcrit issu de l'amplification de l'ARN du patient) au cours de l'hybridation. Il peut s'agir d'oligonucléotides spécifiques de régions différentes de la même séquence cible, ou centrés différemment sur une même région. On utilise avantageusement des probesets comprenant 1-3 sondes, qui peuvent être chevauchantes ou non, en tout ou en partie, et qui sont spécifiques de la même molécule cible. On peut également utiliser des couples de sondes, dont un membre est parfaitement apparié à la séquence cible, et un autre présente un mésappariement, permettant ainsi d'estimer le bruit de fond. Les sondes peuvent être conçues pour s'hybrider à une région d'un exon ou d'un intron, ou à une région de jonction de type exon-exon, exon-intron ou intron-intron. Ainsi, les sondes permettent de mettre en évidence et de distinguer différentes formes d'épissage d'un gène.

Dans un mode de mise en oeuvre préféré, on utilise des sondes dont la séquence comprend tout ou une partie d'une séquence nucléique choisie parmi SEQ ID NOs : 1 à 1754 ou d'une séquence complémentaire de celles-ci. Dans un mode préféré, on utilise des sondes nucléiques ayant une longueur comprise entre 15 et 50 bases, plus préférentiellement entre 15 et 40 bases, et dont la séquence est identique à un fragment d'une séquence choisie parmi SEQ ID NO : 1 à 1754 ou d'une séquence complémentaire de celles-ci. La sonde peut également être conçue dans l'orientation opposée.

Dans un mode particulièrement préféré on utilise des probesets, c'est-à-dire des jeux de 1-3 sondes comprenant chacune une partie, chevauchante ou non, de la même séquence nucléique choisie parmi SEQ ID NOs : 1 à 1754 ou de son brin complémentaire.

Les sondes peuvent être synthétisées préalablement puis déposées sur le support, ou synthétisées directement in situ, sur le support, selon des méthodes connues en soi de l'homme du métier. Les sondes peuvent également être fabriquées par des techniques génétiques, par exemple par amplification, recombinaison, ligation, etc.

Les sondes ainsi définies constituent un autre objet de la présente demande, ainsi que leurs utilisations (essentiellement *in vitro*) pour la détection de la maladie d'Alzheimer chez un sujet.

L'hybridation peut être réalisée dans des conditions classiques, connues de l'homme du métier et ajustables par celui-ci (Sambrook, Fritsch, Maniatis (1989) Molecular Cloning, Cold Spring Harbor Laboratory Press). En particulier, l'hybridation peut être réalisée dans des conditions de stringence élevée, moyenne ou faible, selon le niveau de sensibilité recherché, la quantité de matériel disponible, etc. Par exemple, des conditions appropriées d'hybridation incluent une température comprise entre 55 et 63°C pendant 2 à 18 heures. D'autres conditions d'hybridation, adaptées à des supports de haute densité, sont par exemple une température d'hybridation entre 45 et 55°C. Après l'hybridation, différents lavages peuvent être réalisés pour éliminer les molécules non-hybridées, typiquement dans des tampons SSC comprenant du SDS, tels que un tampon comprenant 0,1 à 10 X SSC et 0,5-0,01% SDS. D'autres tampons de lavage contenant du SSPE, du MES, du NaCl ou du EDTA peuvent également être utilisés.

Dans un mode de mise en oeuvre typique, les acides nucléiques (ou les puces ou supports) sont pré-hybridés dans un tampon d'hybridation (Rapid Hybrid Buffer, Amersham) contenant typiquement 100 µg/ml d'ADN de sperme de saumon à 65°C pendant 30 min. Les acides nucléiques de l'échantillon sont ensuite mis en contact avec les sondes (typiquement appliqués sur le support ou la puce) à 65°C pendant 2 à 18 heures. De préférence, les acides nucléiques de l'échantillon sont marqués au préalable, par tout marquage connu (radioactif, enzymatique, fluorescent, luminescent, etc.). Les supports sont ensuite lavés dans un tampon 5X SSC, 0,1% SDS à 65°C pendant 30 min, puis dans un tampon 0.2X SSC, 0,1% SDS. Le profil d'hybridation est analysé selon des techniques classiques, comme par exemple en mesurant le marquage sur le support au moyen d'un instrument adapté (par exemple InstantImager, Packard Instruments). Les conditions de l'hybridation peuvent naturellement être ajustées par l'homme du métier, par exemple en modifiant la température d'hybridation et/ou la concentration saline du tampon ainsi que par ajout de substances auxiliaires comme le formamide ou de l'ADN simple brin.

Un objet particulier de l'invention réside ainsi dans une méthode pour détecter la présence ou le risque de développer la maladie d'Alzheimer chez un mammifère, ou pour évaluer la réponse à un traitement contre la maladie d'Alzheimer, comprenant la mise en contact, dans des conditions permettant une hybridation entre séquences complémentaires, des acides nucléiques issus d'un échantillon de sang du mammifère et d'un ensemble de sondes spécifique des molécules cibles identifiées précédemment pour obtenir un profil d'hybridation, le profil d'hybridation étant caractéristique de la présence ou du risque de développer la maladie d'Alzheimer chez ce mammifère, ou de l'efficacité du traitement.

Un objet particulier de l'invention réside ainsi dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes spécifique des molécules cibles suivantes au moins:
a) les acides nucléiques comprenant les séquences de l'un des panels 1 à 16 définis précédemment, ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b), pour obtenir un profil d'hybridation, le profil d'hybridation étant caractéristique de la présence de la maladie d'Alzheimer chez ce mammifère.

Dans des modes particuliers de mise en oeuvre, les procédés de l'invention utilisent en outre d'autres molécules cibles et/ou d'autres sondes, notamment les sous-ensembles de molécules cibles mentionnés dans la présente demande.

Ainsi, un autre objet particulier de l'invention réside dans une méthode pour détecter la présence ou le risque de développer la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes spécifique d'au moins deux molécules distinctes choisies parmi les cibles suivantes:
a) les acides nucléiques comprenant les séquences représentées dans SEQ ID NOs: 1 à 1754 ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b), pour obtenir un profil d'hybridation, le profil d'hybridation étant caractéristique de la présence de la maladie d'Alzheimer chez ce mammifère.

Un autre objet particulier de l'invention réside dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins une sonde comprenant tout ou une partie de chacune des séquences nucléiques de l'un des panels 1-16 ou de leur séquence complémentaire pour obtenir un profil d'hybridation, le profil d'hybridation étant caractéristique de la présence de la maladie d'Alzheimer chez ce mammifère.

Comme expliqué plus en détails dans la partie expérimentale, un ensemble préféré selon l'invention comprend au moins les sondes suivantes :
- de 1 à 3 sondes, chevauchantes ou non, comprenant chacune tout ou une partie de la séquence SEQ ID NO : 34 ou de sa séquence complémentaire ;
- de 1 à 3 sondes, chevauchantes ou non, comprenant chacune tout ou une partie de la séquence SEQ ID NO : 230 ou de sa séquence complémentaire ;
- de 1 à 3 sondes, chevauchantes ou non, comprenant chacune tout ou une partie de la séquence SEQ ID NO : 341 ou de sa séquence complémentaire ;
- de 1 à 3 sondes, chevauchantes ou non, comprenant chacune tout ou une partie de la séquence SEQ ID NO : 454 ou de sa séquence complémentaire ;
- de 1 à 3 sondes, chevauchantes ou non, comprenant chacune tout ou unepartie de la séquence SEQ ID NO : 664 ou de sa séquence complémentaire ;
- de 1 à 3 sondes, chevauchantes ou non, comprenant chacune tout ou une partie de la séquence SEQ ID NO : 811 ou de sa séquence complémentaire ;
- de 1 à 3 sondes, chevauchantes ou non, comprenant chacune tout ou une partie de la séquence SEQ ID NO : 951 ou de sa séquence complémentaire ;
- de 1 à 3 sondes, chevauchantes ou non, comprenant chacune tout ou une partie de la séquence SEQ ID NO : 1127 ou de sa séquence complémentaire ;
- de 1 à 3 sondes, chevauchantes ou non, comprenant chacune tout ou une partie de la séquence SEQ ID NO : 1136 ou de sa séquence complémentaire ; et
- de 1 à 3 sondes, chevauchantes ou non, comprenant chacune tout ou une partie de la séquence SEQ ID NO : 1752 ou de sa séquence complémentaire.

Comme indiqué précédement, le terme « partie » désigne avantageusement une région de 15 à 50 nucléotides consécutifs.

Il est entendu que l'ensemble peut comprendre, en plus des 10 sondes ou groupes de sondes (probesets) cités, d'autres sondes ou probesets, comprenant par exemple une séquence choisie parmi SEQ ID NO : 1 à 1754 et/ou parmi d'autres séquences.

Ainsi, un autre ensemble préféré selon l'invention comprend, en plus des sondes mentionnées ci-dessus, au moins les sondes supplémentaires suivantes :
- de 1 à 3 sondes, chevauchantes ou non, comprenant chacune tout ou une partie de la séquence SEQ ID NO : 35 ou de sa séquence complémentaire ;
- de 1 à 3 sondes, chevauchantes ou non, comprenant chacune tout ou une partie de la séquence SEQ ID NO : 316 ou de sa séquence complémentaire ;
- de 1 à 3 sondes, chevauchantes ou non, comprenant chacune tout ou une partie de la séquence SEQ ID NO : 593 ou de sa séquence complémentaire ;
- de 1 à 3 sondes, chevauchantes ou non, comprenant chacune tout ou une partie de la séquence SEQ ID NO : 666 ou de sa séquence complémentaire ;
- de 1 à 3 sondes, chevauchantes ou non, comprenant chacune tout ou unepartie de la séquence SEQ ID NO : 855 ou de sa séquence complémentaire ;
- de 1 à 3 sondes, chevauchantes ou non, comprenant chacune tout ou une partie de la séquence SEQ ID NO : 1330 ou de sa séquence complémentaire ; et
- de 1 à 3 sondes, chevauchantes ou non, comprenant chacune tout ou une partie de la séquence SEQ ID NO : 1498 ou de sa séquence complémentaire.

Les panels 1-14 peuvent être définis comme l'ensemble ci-dessus. Les sondes de base constituant ces ensembles sont données dans les exemples.

Un autre objet de l'invention réside dans une méthode pour détecter la présence ou le risque de développer la maladie d'Alzheimer chez un mammifère, comprenant la détection, à partir d'un échantillon de sang du mammifère, d'une variation dans les niveaux d'acides nucléiques complémentaires d'un ensemble de sondes, l'ensemble de sondes comprenant au moins une sonde comprenant tout ou partie de chacune des séquences nucléiques de l'un des panels définis précédemment, une variation étant caractéristique de la présence ou du risque de développer la maladie d'Alzheimer chez ce mammifère.

Le profil d'hybridation peut être comparé à un ou plusieurs profils de référence, notamment un profil de référence caractéristique de sujets sains et/ou de sujets atteints de la maladie d'Alzheimer, la comparaison permettant de déterminer la probabilité que le patient testé soit atteint de la maladie d'Alzheimer. Typiquement, la comparaison est réalisée au moyen de programmes informatiques connus en soi de l'homme du métier.

L'amplification sélective est de préférence réalisée en utilisant une amorce ou une paire d'amorces permettant l'amplification de tout ou partie d'un des acides nucléiques cibles dans l'échantillon, lorsque celui-ci y est présent. L'amorce peut être spécifique d'une séquence cible telle que définie précédemment selon SEQ ID NO : 1 à 1754, ou d'une région flanquant la séquence cible dans un acide nucléique de l'échantillon. L'amorce comprend typiquement un acide nucléique simple-brin, d'une longueur comprise avantageusement entre 5 et 50 bases, de préférence entre 5 et 30. Une telle amorce constitue un autre objet de la présente demande, ainsi que son utilisation (essentiellement *in vitro*) pour la détection de la maladie d'Alzheimer chez un sujet. Les amorces peuvent être conçues pour s'hybrider à une région d'un exon ou d'un intron, ou à une région de jonction de type exon-exon, exon-intron ou intron-intron. Ainsi, les amorces permettent de mettre en évidence et de distinguer différentes formes d'épissage d'un gène.

A cet égard, un autre objet de l'invention réside dans l'utilisation d'une amorce nucléotidique ou d'un ensemble d'amorces nucléotidiques permettant l'amplification de tout ou partie d'un ou, de préférence plusieurs gènes ou ARN comprenant une séquence cible selon SEQ ID NO : 1 à 1754, pour détecter la présence de la maladie d'Alzheimer chez un mammifère, ou pour évaluer la réponse à un traitement contre de la maladie d'Alzheimer chez un mammifère, tout particulièrement chez un être humain.

Un autre objet particulier de l'invention réside dans une méthode pour détecter la présence de la maladie d'Alzheimer chez un mammifère, comprenant la mise en contact, dans des conditions permettant une amplification, des acides nucléiques issus d'un échantillon de sang du mammifère et d'un ensemble d'amorces spécifique d'au moins deux molécules distinctes choisies parmi les cibles suivantes:
a) les acides nucléiques comprenant les séquences représentées dans SEQ ID NOs: 1 à 1754 ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b), pour obtenir un profil d'amplification, le profil d'amplification étant caractéristique de la présence de la maladie d'Alzheimer chez ce mammifère.

### Détection d'une altération dans un polypeptide

Dans un autre mode de réalisation, la méthode comprend la détermination de la présence ou de la quantité (relative) d'un polypeptide codé par un gène tel que défini précédemment. La mise en évidence ou le dosage d'un polypeptide dans un échantillon peut être réalisée par toute technique connue en soi, comme notamment au moyen d'un ligand spécifique, par exemple un anticorps ou un fragment ou dérivé d'anticorps. De préférence, le ligand est un anticorps spécifique du polypeptide, ou un fragment d'un tel anticorps (par exemple un Fab, Fab', CDR, etc.), ou un dérivé d'un tel anticorps (par exemple un anticorps simple-chaîne, ScFv). Le ligand est typiquement immobilisé sur un support, tel qu'une lame, bille, colonne, plaque, etc. La présence ou la quantité du polypeptide cible dans l'échantillon peut être détectée par la mise en évidence d'un complexe entre la cible et le ligand, par exemple en utilisant un ligand marqué, en utilisant un deuxième ligand de révélation marqué, etc. Des techniques immunologiques utilisables et bien connues sont les techniques ELISA, RIA, etc. Si nécessaire, la quantité de polypeptide détectée peut être comparée à une valeur de référence, par exemple une valeur médiane ou moyenne observée chez des patients qui ne sont pas atteints de la maladie d'Alzheimer, ou à une valeur mesurée en parallèle dans un échantillon témoin. Ainsi, il est possible de mettre en évidence une variation de niveaux d'expression.

Des anticorps spécifiques des polypeptides cibles peuvent être produits par des techniques conventionnelles, notamment par immunisation d'un animal non-humain avec un immunogène comprenant le polypeptide (ou un fragment immunogène de celui-ci), et récupération des anticorps (polyclonaux) ou des cellules productrices (pour produire des monoclonaux). Des techniques de production d'anticorps poly- ou monoclonaux, de fragments ScFv, d'anticorps humains ou humanisés sont décrites par exemple dans Harlow et al., Antibodies: A laboratory Manual, CSH Press, 1988 ; Ward et al., Nature 341 (1989) 544 ; Bird et al., Science 242 (1988) 423 ; WO94/02602 US5,223,409 ; US5,877,293 ; WO93/01288. L'immunogène peut être fabriqué par synthèse, ou par expression, dans un hôte approprié, d'un acide nucléique cible tel que défini ci-avant. Un tel anticorps, monoclonal ou polyclonal, ainsi que ses dérivés ayant la même spécificité antigénique, constituent également un objet de la présente demande, de même que leur utilisation pour détecter la maladie d'Alzheimer.

Des modifications de l'expression et/ou de la structure des protéines peuvent aussi être détectées au moyen des techniques connues en soi de l'homme du métier et impliquant la spectroscopie de masse, plus généralement regroupées sous le nom d'analyse protéomique, afin de détecter des signatures spécifiques du sang des patients atteints de la maladie d'Alzheimer.

### Mise en oeuvre du procédé

La méthode de l'invention est applicable à tout échantillon biologique du mammifère testé, en particulier tout échantillon comportant des acides nucléiques ou des polypeptides. On peut citer avantageusement un échantillon de sang, plasma, plaquette, salive, urine, selles, etc., plus généralement tout tissu, organe ou, avantageusement, fluide biologique comportant des acides nucléiques ou des polypeptides.

Dans un mode de mise en oeuvre préféré et particulièrement avantageux, l'échantillon est un échantillon dérivé du sang, par exemple un échantillon de sang, de sérum ou de plasma. L'invention découle en effet de l'identification de marqueurs sanguins de la maladie d'Alzheimer, et permet donc une détection de cette pathologie sans biopsie tissulaire, mais uniquement à partir de prélèvements sanguins.

L'échantillon peut être obtenu par toute technique connue en soi, par exemple par prélèvement, par des techniques non invasives, à partir de collections ou banques d'échantillons, etc. L'échantillon peut par ailleurs être pré-traité pour faciliter l'accessibilité des molécules cibles, par exemple par lyse (mécanique, chimique, enzymatique, etc.), purification, centrifugation, séparation, etc. L'échantillon peut également être marqué, pour faciliter la détermination de la présence des molécules cibles (marquage fluorescent, radioactif, luminescent, chimique, enzymatique, etc.). Les acides nucléiques de l'échantillon peuvent par ailleurs être séparés, traités, enrichis, purifiés, rétro-transcrits, amplifiés, fragmentés, etc. Dans un mode particulier, les acides nucléiques de l'échantillon sont les ou des ARN, notamment les ou des ARNm de l'échantillon. Dans un mode tout particulier, les acides nucléiques sont le produit d'amplification des ARN, notamment des ARNm ; ou les/des ADNc préparés à partir des ARN, notamment des ARNm de l'échantillon.

Dans un mode de mise en oeuvre préféré, l'échantillon biologique est un échantillon de sang total, c'est-à-dire n'ayant pas subi d'étape de séparation, qui peut être éventuellement dilué.

L'invention est applicable à tout mammifère, de préférence les humains. La méthode de l'invention est particulièrement utile pour la détection de la maladie d'Alzheimer, notamment de la présence ou du degré de sévérité / avancement de la maladie d'Alzheimer chez l'être humain. Ainsi, les données fournies dans les exemples montrent que l'invention permet de détecter la présence de la maladie d'Alzheimer avec une sensibilité supérieure à 75% et une spécificité supérieure à 75 %. Ces résultats sont particulièrement remarquables. En effet, il est connu que le taux de faux positifs dans le diagnostic actuel de patients sains sur la base d'examens cliniques est de l'ordre de 15 à 30% et le taux de faux positifs dans le diagnostic actuel de patients AD est de 15%, sur la base d'examens cliniques multiples. Le diagnostic certain ne peut être établi que post mortem à partir d'une autopsie du cerveau.

Un objet particulier de la présente demande concerne une méthode pour détecter la présence ou l'évolution de la maladie d'Alzheimer chez un sujet humain, comprenant la détermination combinée de la présence (ou de l'absence ou de la quantité (relative)), dans un échantillon biologique du sujet humain, de molécules cibles choisies parmi:
a) les acides nucléiques comprenant une séquence choisie parmi SEQ ID NO: 1 à 1754 ou un fragment de celles-ci ayant au moins 15, de préférence au moins 16, 17, 18, 19, 20, 25 ou 30 bases consécutives,
b) les acides nucléiques ayant une séquence complémentaire d'une séquence selon a), et
c) les polypeptides codés par les acides nucléiques selon a) ou b).

De préférence, la méthode comprend la détermination combinée de la présence, absence ou quantité de 5, 10, 20, 30, 40, 50 ou 60 molécules cibles telles que définies ci-dessus.

Un autre objet particulier de la présente demande concerne une méthode pour détecter la présence ou l'évolution de la maladie d'Alzheimer chez un sujet humain, comprenant la mise en contact d'un échantillon biologique du sujet contenant des acides nucléiques avec un produit comprenant un support sur lequel sont immobilisés des acides nucléiques comprenant une séquence complémentaire et/ou spécifique d'une ou, de préférence, plusieurs molécules cibles choisies parmi (i) les acides nucléiques comprenant une séquence choisie parmi SEQ ID NO: 1 à 1754 ou un fragment de celles-ci ayant au moins 15, de préférence au moins 16, 17, 18, 19, 20, 25 ou 30 bases consécutives et (ii) les acides nucléiques ayant une séquence complémentaire d'une séquence selon (i), et le profil indiquant la présence, le degré de sévérité / avancement ou le risque de développer la maladie d'Alzheimer chez ledit sujet humain. De préférence, le produit comprend des acides nucléiques distincts comprenant une séquence complémentaire et/ou spécifique d'au moins 5, 10, 20, 30, 40, 50, 60 ou plus gènes ou ARNs différents tels que mentionnés ci-dessus.

Un autre objet de la présente demande concerne un produit comprenant un support sur lequel sont immobilisés des acides nucléiques comprenant une séquence complémentaire et/ou spécifique d'une ou, de préférence, plusieurs molécules cibles choisies parmi (i) les acides nucléiques comprenant une séquence choisie parmi SEQ ID NO: 1 à 1754 ou un fragment de celles-ci ayant au moins 15, de préférence au moins 16, 17, 18, 19, 20, 25 ou 30 bases consécutives et (ii) les acides nucléiques ayant une séquence complémentaire d'une séquence selon (i). De préférence, le produit comprend des acides nucléiques distincts comprenant une séquence complémentaire et/ou spécifique d'au moins 5, 10, 20, 30, 40, 50, 60 ou plus gènes ou ARN tels que définis précédemment.

Un autre objet de la présente demande concerne un produit comprenant un support sur lequel est immobilisé au moins un, de préférence plusieurs, acides nucléiques comprenant une séquence choisie parmi SEQ ID NO: 1 à 1754, ou un analogue fonctionnel de celles-ci. De préférence, le produit comprend au moins 5, 10, 20, 30, 40, 50, 60 ou plus acides nucléiques différents choisis parmi les acides nucléiques mentionnés ci-dessus.

Un autre objet particulier de l'invention concerne un produit comprenant un support sur lequel est immobilisé un ensemble de sondes, l'ensemble contenant au moins une sonde comprenant tout ou une partie de chacune des séquences nucléiques cibles suivantes, ou de leur brin complémentaire : SEQ ID Nos : 34, 230, 341, 454, 664, 811, 951, 1127 ; 1136 et 1752.

Un autre objet particulier de l'invention concerne un produit comprenant un support sur lequel est immobilisé un ensemble de sondes, l'ensemble contenant au moins une sonde comprenant tout ou une partie de chacune des séquences nucléiques cibles suivantes, ou de leur brin complémentaire : SEQ ID Nos : 34, 35 ; 230, 316 ; 341, 454, 593 ; 664, 666 ; 811 ; 855 ; 951 ; 1127 ; 1136 ; 1330; 1498 et 1752.

Un produit préféré de l'invention comprend un support sur lequel est immobilisé au moins un ensemble de sondes d'acides nucléiques distinctes comprenant une séquence complémentaire et/ou spécifique des acides nucléiques de l'un des panels 1 à 14 définis dans le tableau 1.

Typiquement, les sondes sont choisies parmi SEQ ID : 1755-6532.

L'invention concerne encore un kit comprenant un compartiment ou conteneur comprenant au moins 5, 10, 20, 30, 40, 50, 60 ou plus acides nucléiques différents choisis parmi les acides nucléiques définis précédemment.

L'invention concerne également un kit comprenant un produit tel que défini précédemment et des réactifs pour une réaction d'hybridation.

L'invention concerne encore l'utilisation d'un produit ou kit défini ci-dessus pour la détection *in vitro* ou *ex vivo* de la présence de la maladie d'Alzheimer, ou de la réponse à un traitement de la maladie d'Alzheimer chez un sujet.

Un autre objet de la présente demande concerne un produit comprenant un support sur lequel est immobilisé au moins un ligand d'un polypeptide codé par un acide nucléique cible tel que défini ci-dessus, c'est-à-dire un acide nucléique comprenant une séquence choisie parmi SEQ ID NO: 1 à 1754, un fragment distinctif de celles-ci ayant au moins 15, de préférence au moins 16, 17, 18, 19, 20, 25 ou 30 bases consécutives, un acide nucléique ayant une séquence complémentaire de celles-ci ou un analogue fonctionnel de celles-ci. De préférence, le produit comprend au moins 5, 10, 20, 30, 40, 50, 60 ou plus ligands de polypeptides différents choisis parmi les polypeptides mentionnés ci-dessus.

Le support peut être tout support solide ou semi-solide présentant au moins une surface, plane ou non (c'est-à-dire en 2 ou 3 dimensions), permettant l'immobilisation d'acides nucléiques ou de polypeptides. De tels supports sont par exemple une lame, bille, membrane, filtre, colonne, plaque, etc. Ils peuvent être réalisés en tout matériau compatible, comme notamment du verre, silice, plastique, fibre, métal, polymère, polystyrène, téflon, etc. Les réactifs peuvent être immobilisés sur la surface du support par des techniques connues, ou, dans le cas des acides nucléiques, synthétisés directement in situ sur le support. Des techniques d'immobilisation incluent l'adsorption passive (Inouye et al., J. Clin. Microbiol. 28 (1990) 1469), la liaison covalente. Des techniques sont décrites par exemple dans WO90/03382, WO99/46403. Les réactifs immobilisés sur le support peuvent être ordonnés selon un schéma pré-établi, pour faciliter la détection et l'identification des complexes formés, et selon une densité variable et adaptable.

Dans un mode de mise en oeuvre, le produit de l'invention comprend une pluralité d'oligonucléotides synthétiques, d'une longueur comprise entre 5 et 100 bases, spécifiques d'un ou plusieurs gènes ou ARNs tels que définis précédemment.

Les produits de l'invention comprennent typiquement des molécules contrôle, permettant d'étalonner et/ounormaliser les résultats.

Un autre objet de la présente demande concerne un produit comprenant un support sur lequel sont immobilisés des acides nucléiques comprenant tout ou partie des séquences choisies parmi SEQ ID NO : 1755-6532. Ces séquences représentent des sondes spécifiques des SEQ ID NO : 1 à 1754. Un tel produit pourra avantageusement incorporer des acides nucléiques choisis pour leur caractère non discriminant de population de patients atteints de la maladie d'Alzheimer, de tels acides nucléiques servant de contrôles de normalisation du produit. Ces acides nucléiques peuvent correspondre à tout ou partie des séquences choisies parmi SEQ ID NO : 1755-6532.

Un autre objet de la présente demande concerne un kit comprenant un compartiment ou conteneur comprenant au moins un, de préférence plusieurs, acides nucléiques comprenant une séquence complémentaire et/ou spécifique d'un ou plusieurs gènes ou ARNs tels que définis précédemment et/ou un, de préférence plusieurs ligands d'un ou plusieurs polypeptides tels que définis précédemment. De préférence, le produit comprend au moins 5, 10, 20, 30, 40, 50, 60 ou plus acides nucléiques et/ou ligands différents choisis parmi les acides nucléiques et ligands mentionnés ci-dessus. Dans un mode particulier de mise en oeuvre, le produit comprend chacun des acides nucléiques de séquence SEQ ID NO: 1 à 1754 ou un ligand pour chacun des polypeptides cibles tels que définis ci-dessus. Dans un autre mode particulier de mise en oeuvre, le produit comprend chacun des acides nucléiques de séquence SEQ ID NO : 1755-6532. Le kit peut comprendre par ailleurs des réactifs pour une réaction d'hybridation ou immunologique, ainsi que, le cas échéant, des contrôles et/ou instructions.

Un autre objet de l'invention concerne l'utilisation d'un produit ou kit tel que défini ci-dessus pour la détection de la maladie d'Alzheimer chez un sujet mammifère, de préférence un sujet humain.

Un autre objet de l'invention concerne un acide nucléique de séquence choisie parmi SEQ ID NO : 1 à 1754, ou un fragment distinctif de celles-ci comprenant au moins 15 bases consécutives, de préférence au moins 16, 17, 18, 19, 20, 25 ou 30, ou un acide nucléique ayant une séquence complémentaire de celles-ci, ou un analogue fonctionnel de celles-ci. L'invention concerne également un vecteur de clonage ou d'expression comportant ces acides nucléiques, ainsi que toute cellule recombinante comprenant un tel vecteur ou acide nucléique.

Un autre objet de l'invention concerne l'utilisation d'un acide nucléique comprenant une séquence choisie parmi SEQ ID NO : 1 à 1754, ou un fragment distinctif de celles-ci comprenant au moins 15 bases consécutives, de préférence au moins 16, 17, 18, 19, 20, 25 ou 30, ou un acide nucléique ayant une séquence complémentaire de celles-ci, ou un analogue fonctionnel de celles-ci, pour la détection (essentiellement *in vitro*) de la maladie d'Alzheimer chez un sujet mammifère.

Selon un exemple particulier de mise en oeuvre de l'invention, on prélève un échantillon de sang d'un mammifère à tester. L'échantillon de sang est éventuellement traité de manière à rendre les acides nucléiques plus accessibles, et ceux-ci sont marqués. Les acides nucléiques sont ensuite appliqués sur un produit tel que défini ci-avant et le profil d'hybridation est déterminé, permettant de diagnostiquer la présence ou non de la maladie d'Alzheimer chez le sujet. La méthode de l'invention est simple, pratiquée *ex vivo,* et permet la détection précoce de la maladie d'Alzheimer à partir d'un échantillon de sang.

Il est entendu que toute technique équivalente peut être utilisée dans le cadre de la présente demande pour déterminer la présence d'une molécule cible.

D'autres aspects et avantages de la présente invention apparaîtront à la lecture des exemples qui suivent, qui doivent être considérés comme illustratifs et non limitatifs.

### LEGENDE DES FIGURES

**Figure 1.** Configuration des sondes présentes sur le microarray GWSA. A) Les sondes exons de type B, F et T et les sondes de jonction de type C, D et E permettent de couvrir de façon optimale les événements d'épissage. B) Des sondes capables de couvrir tout saut d'exon unique sont également présentes avec la même configuration. De même, des sondes de jonction exon - exon (X), exon - intron (Z) et intron -exon (Y) sont également présentes.

### LEGENDE DES TABLEAUX

**Tableau 1.** Description des séquences SEQ ID NO : 1 à 6532.
**Tableau 2.** Liste des probesets de chacun des panels 1 à 14.

### Exemple 1: Identification de marqueurs sériques de la maladie d'Alzheimer

### 1.1. Caractéristiques des échantillons biologiques

Les exemples présentés ci après ont été réalisés initialement à partir de 177 ou 100 parmi les 177 échantillons sanguins (2.5 ml de sang total, prélevé dans deux tubes PaxGene). Ces échantillons regroupaient 90 patients diagnostiqués d'une maladie d'Alzheimer probable selon les critères du DSM IV. Ces patients correspondaient à un age moyen de 78,08 ans (écart-type : 6,67 ans). Ces patients présentaient un score MMSE (Mini-Mental State Examination) inférieur à 27 (score moyen de 17,16 ; écart-type de 6,04. Par ailleurs 87 sujets, d'âge comparable aux patients AD, déclarés non déments après un examen clinique ont également été recrutés (age moyen de 69,71 ans avec un écart-type de 6,53 ans ; MMSE moyen de 29,31 avec un écart-type de 0,97).

### 1.2. Extraction des ARN totaux de l'échantillon sanguin

Les prélèvements sanguins ont été collectés directement dans des tubes PAXGene^{™} Blood RNA (PreAnalytix, Hombrechtikon, CH). Après l'étape de prélèvement de l'échantillon sanguin et afin d'obtenir une lyse totale des cellules, les tubes ont été laissés à température ambiante pendant 4 h puis conservés à -20°C ou -80°C jusqu'à l'extraction du matériel biologique. Plus précisément, dans ce protocole, les ARN totaux ont été extraits à l'aide des kits PAXGene Blood RNA® (PreAnalytix) en respectant les recommandations du fabriquant. Brièvement, les tubes ont été centrifugés (10 min, 3000 g) afin d'obtenir un culot d'acides nucléiques. Ce culot a été lavé et repris dans un tampon contenant de la protéinase K nécessaire à la digestion des protéines (10 min à 55°C). Une nouvelle centrifugation (3 min, 14 000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen, Hilden, Allemagne). La qualité et la quantité des ARN totaux extraits sont évaluées par réalisation d'électrophorégrammes à l'aide du Bioanalyser 2100 d'Agilent en utilisant le kit RNA 6000 NanoChip (Agilent Technologies, Santa Clara, CA). Seuls les échantillons remplissant les critères de qualité ont été utilisés pour des analyses ultérieures.

### 1.3. Le micro-array GWSA (Genome Wide SpliceArray)

La détection et la quantification de l'expression exhaustive des transcrits par microarray nécessite l'utilisation d'une configuration de sondes particulière. Tout couple ARN messager de référence / variant d'épissage peut être modélisé en tant que Isoforme longue / Isoforme courte (Figure lA). Ainsi, un variant d'épissage associé à un saut d'exon sera l'isoforme courte par rapport au variant de référence. Un variant d'épissage comportant un nouvel exon ou une rétention d'intron sera l'isoforme longue par rapport au variant de référence. Les autres événements d'épissage alternatifs (utilisations de sites cryptiques 5' ou 3' d'épissage) peuvent également être modélisés de la même manière.

Le jeu de sondes nécessaire à la mesure de l'expression de variants d'épissage est également indiqué sur la figure 1A. Ce jeu est composé de trois sondes « exoniques » traditionnelles F, T et B et de trois sondes de jonction de type exon-exon ou exon-intron C, D et E. Les sondes F et T mesurent l'expression des deux isoformes, les sondes B, C et D l'expression de l'isoforme longue et la sonde E l'expression de la forme courte.

Afin de concevoir toutes ces sondes, il est nécessaire d'identifier les événements d'épissage correspondant aux gènes humains, puis les régions « cibles » à partir desquelles seront dessinées les sondes. Les séquences cibles correspondant aux sondes de jonction C, D et E sont définies par une longueur de 30 nucléotides, 15 nucléotides de part et d'autre de la jonction. Il est ainsi possible de « couvrir » toute jonction par des sondes de 25 nucléotides par exemple de type : 11/14, 12/13, 13/12 (le signe / représentant la zone de jonction).

La puce GWSA est un microarray capable de fournir une couverture très complète de l'expression du génome humain en prenant en compte l'existence des variants d'épissage. 20,649 gènes humains ont été sélectionnés puis analysés pour identifier des événements d'épissage connus et potentiels associés. Plus de 90% de ces gènes pouvaient être associés à de tels événements. Des sondes telles que décrites ci-dessus ont été dessinées à partir des quelques 140,000 événement d'épissage identifiés. Par ailleurs, l'utilisation de sondes spécifiques d'événements d'épissages correspondant à des sauts uniques d'exons permet également de prédire l'existence de tels événements (Figure 1B). De plus, l'incorporation de sondes de jonction couvrant chaque région exon - exon, exon - intron et intron -exon de chaque gène permet également de caractériser des évenements d'épissages affectant les extrémités 5' et 3' des exons (Figure 1B). Trois sondes différentes ont été conçues par séquence cible et regroupées en « Probe Set ». Les valeurs d'expression des sondes individuelles sont consolidées en une valeur de Probe Set. Parfois, le Probe Set ne contiendra que 2 voire 1 sonde si les restrictions sur la séquence cible étaient trop contraignantes. Le GWSA est un microarray à façon construit sur la plateforme GeneChip® d'Affymetrix à une résolution de 5 microns. Le GWSA a passé un contrôle qualité selon les normes MAQC (Microarray Quality Control ; Shi et al., Nat Biotechnol. 2006; 24(9): 1151-61).

### 1.4. Amplification de l'ARN

50 ng d'ARN total ont servi de matrice pour la synthèse de cibles à l'aide du kit WT-Ovation™ Pico RNA Amplification System (NuGen, San Carlos, CA). Le module FL-Ovation™ cDNA Biotin Module (NuGen, San Carlos, CA) a ensuite été utilisé pour réaliser la fragmentation de 5 µg de cDNA amplifié ainsi que le marquage à la biotine. Les différentes étapes ont été réalisées en suivant les instructions du fabricant. Chaque série d'amplification/fragmentation/marquage contenait autant d'échantillons correspondant à des patients AD et contrôle.
La qualité et la quantité des ADN complémentaires extraits sont évaluées par réalisation d'électrophorégrammes à l'aide du Bioanalyser 2100 d'Agilent en utilisant le kit RNA 6000 NanoChip (Agilent Technologies, Santa Clara, CA).

### 1.5. Hybridation sur la lame GWSA

5 µg de cDNA amplifiés et marqués à la biotine sont utilisés par hybridation. Les méthodes standard recommandées par Affymetrix (Affymetrix, Santa Clara, CA) ont été appliquées pour l'hybridation des cibles sur le microarray GWSA. Les puces à ADN ont ensuite été lavées et l'hybridation spécifique révélée en suivant les recommandations d'Affymetrix. Les signaux d'hybridation ont été détectés à l'aide du scanner GeneChip^{R}3000 7G.

### 1.6. Extraction des données et normalisation

Les fichiers .CEL obtenus après scanning des lames ont ensuite été importés dans Partek Genomic Suites^{™} (Partek Incorporated, St Louis, MI) pour une normalisation quantile des lames, un ajustement du bruit de fond en fonction la composition en GC des oligonucléotides sondes, une correction du bruit de fond par RMA et une consolidation des valeurs d'expression mesurée pour chaque oligonucléotide en probe set (1455 607 PS). Les données ont ensuite été filtrées par rapport aux valeurs d'expression des probe sets, le seuil de niveau d'expression a été fixé à 6.2 (échelle log 2) en se basant sur la fréquence de distribution des valeurs d'expression sur l'ensemble des lames (361 299 PS) et par rapport à la variance, le seuil a été fixé à 0.3 (299 577 PS).

### Exemple 2. Sélection de signatures

Un groupe d'apprentissage de signature(s) discriminante(s) a été utilisé pour des analyses de clustering supervisé à l'aide d'algorithmes de classification binaire et des validations croisées (cross validation) avec des partitions de 5 ou 10 (Partek Genomic Suites^{™}). Des tests non paramétriques (1-way ANOVA) ont été réalisés pour l'analyse des valeurs d'expression obtenues par probe set et servent d'outil de filtrage pour la sélection de variables qui intégreront la signature. Les modèles de classification optimaux par groupe d'apprentissage sont ensuite appliqués au groupe test pour l'identification des performances de la signature à savoir, la spécificité (% de bon classement des patients contrôles) et la sensibilité (% de bon classement des patients AD).

Les modèles ont été testés, les performances de chaque modèle ont été évaluées sur l'ensemble des groupes d'apprentissage. 14 signatures présentant les moyennes de performances les plus élevées ont été sélectionnées pour discriminer entre les patients atteints de la maladie d'Alzheimer et les patients contrôles non déments. La liste des marqueurs composant ces panels 1-14 est donnée dans le tableau 2. Les performances de ces panels dans les premières études sont regroupées dans le tableau ci-dessous :

| Modèles - panel | AD% | CT% | Performances | PPV | NPV |
|---|---|---|---|---|---|
| AD vs CT-1-240v | 76,90% | 79,60% | 78,30% | 78,43% | 78,18% |
| AD vs CT-2-220v | 73,10% | 79,60% | 76,42% | 77,55% | 75,44% |
| AD vs CT-3-1460v | 75,00% | 77,80% | 76,42% | 76,47% | 76,36% |
| AD vs CT-4-140v | 73,10% | 77,80% | 75,47% | 76,00% | 75,00% |
| AD vs CT-5-160v | 73,10% | 75,90% | 74,53% | 74,51% | 74,55% |
| AD vs CT-6-80v | 73,10% | 75,90% | 74,53% | 74,51% | 74,55% |
| AD vs CT-7-120v | 71,20% | 75,90% | 73,58% | 74,00% | 73,21% |
| AD vs CT-8-240v | 80,80% | 77,80% | 79,25% | 77,80% | 80,80% |
| AD vs CT-9-300v | 78,80% | 77,80% | 78,30% | 77,37% | 79,21% |
| AD vs CT-10-170v | 76,90% | 79,60% | 78,30% | 78,40% | 78,16% |
| AD vs CT-11-130v | 76,90% | 75,90% | 76,42% | 75,45% | 77,33% |
| AD vs CT-12-150v | 76,90% | 74,10% | 75,47% | 74,09% | 76,91% |
| AD vs CT-13-120v | 75,00% | 75,90% | 75,47% | 74,98% | 75,92% |
| AD vs CT-14-250v | 75,00% | 75,90% | 75,47% | 74,98% | 75,92% |

### Exemple 3. Mise en évidence d'un profil d'expression permettant de discriminer les patients Contrôles des patients atteints de la maladie d'Alzheimer (AD) à partir d'échantillons sanguins

L'expression de l'ensemble du transcriptome humain, représentant environ 21,000 gènes, a été analysée et comparée entre des patients AD et CND (contrôles) à l'aide du microarray GWSA. L'ensemble des analyses mentionnées dans l'exemple 2 a permis de mettre en évidence un ensemble de 1754 séquences cibles pertinentes (cf tableau 1, SEQ ID Nos: 1-1754).

Les inventeurs ont ensuite étudié l'expression simultanée de probesets associés à des sous-ensembles de ces 1754 séquences pour obtenir des profils d'expression spécifiques de la maladie d'Alzheimer.

Les performances de ces signatures ont été évaluées sur un ensemble TEST (set de validaton) d'individus n'ayant pas participé à la définition de cette signature et recrutés dans une étude en aveugle. Les valeurs des performances sont décrites dans l'exemple 2.

L'analyse des signatures au niveau du gène a révélé que 10 gènes sont communs à l'ensemble des 14 panels. La liste des gènes correspondants, est donnée ci-dessous (panel 15).

| **Entrez ID** | **Symbol** | **Description** |
|---|---|---|
| 10308 | ZNF267 | zinc finger protein 267 |
| 1462 | CSPG2 | chondroitin sulfate proteoglycan 2 (versican) |
| 22832 | KIAA1009 | kiaa1009 |
| 2591 | GALNT3 | udp-n-acetyl-alpha-d-galactosamine |
| 3717 | JAK2 | janus kinase 2 (a protein tyrosine kinase) |
| 51086 | TNN13K | tnni3 interacting kinase |
| 54851 | ANKRD49 | ankyrin repeat domain 49 |
| 597 | BCL2A1 | bcl2-related protein a1 |
| 6093 | ROCK1 | rho-associated, coiled-coil containing protein kinase 1 |
| 9976 | CLEC2B | c-type lectin domain family 2, member b |

Un exemple préféré d'ensemble de sondes pour la détection de la maladie d'Alzheimer comprend ainsi:
- de 1 à 3 sondes, chevauchantes ou non, comprenant chacune tout ou une partie de la séquence SEQ ID NO : 34 ou de sa séquence complémentaire ;
- de 1 à 3 sondes, chevauchantes ou non, comprenant chacune tout ou une partie de la séquence SEQ ID NO : 230 ou de sa séquence complémentaire ;
- de 1 à 3 sondes, chevauchantes ou non, comprenant chacune tout ou une partie de la séquence SEQ ID NO : 341 ou de sa séquence complémentaire ;
- de 1 à 3 sondes, chevauchantes ou non, comprenant chacune tout ou une partie de la séquence SEQ ID NO : 454 ou de sa séquence complémentaire ;
- de 1 à 3 sondes, chevauchantes ou non, comprenant chacune tout ou une partie de la séquence SEQ ID NO : 664 ou de sa séquence complémentaire ;
- de 1 à 3 sondes, chevauchantes ou non, comprenant chacune tout ou une partie de la séquence SEQ ID NO : 811 ou de sa séquence complémentaire ;
- de 1 à 3 sondes, chevauchantes ou non, comprenant chacune tout ou une partie de la séquence SEQ ID NO : 951 ou de sa séquence complémentaire ;
- de 1 à 3 sondes, chevauchantes ou non, comprenant chacune tout ou une partie de la séquence SEQ ID NO : 1127 ou de sa séquence complémentaire ;
- de 1 à 3 sondes, chevauchantes ou non, comprenant chacune tout ou une partie de la séquence SEQ ID NO : 1136 ou de sa séquence complémentaire ; et
- de 1 à 3 sondes, chevauchantes ou non, comprenant chacune tout ou une partie de la séquence SEQ ID NO : 1752 ou de sa séquence complémentaire.

### Exemple 4. Mise en évidence d'un profil d'expression permettant de discriminer les patients Contrôles des patients atteints de la maladie d'Alzheimer (AD) à partir d'échantillons sanguins

L'analyse des signatures au niveau du gène a permis de sélectionner 17 marqueurs communs aux 5 panels présentant les meilleurs performances. La liste de ces 17 marqueurs (qui inclut les 10 marqueurs de l'exemple 3), est donnée ci-dessous (panel 16).

| **17 PS ADvsC** | **SEQID** |
|---|---|
| 10308.001.2-T | 34 |
| 10308.006.1-F | 35 |
| 1462.002.1-B | 230 |
| 2123.001.1-T | 316 |
| 22832.016.1-B | 341 |
| 2591.003.1-T | 454 |
| 3146.000.2-X | 593 |
| 3717.000.10-X | 664 |
| 3717.000.23-X | 666 |
| 51086.001.1-B | 811 |
| 51426.010.1-B | 855 |
| 54851.003.1-F | 951 |
| 597.000.1-Z | 1127 |
| 6093.031.1-B | 1136 |
| 6672.027.1-T | 1330 |
| 830.000.5-Z | 1498 |
| 9976.003.1-F | 1752 |

Un autre exemple préféré d'ensemble de sondes pour la détection de la maladie d'Alzheimer comprend ainsi:
- de 1 à 3 sondes, chevauchantes ou non, comprenant chacune tout ou une partie de la séquence SEQ ID NO : 34 ou de sa séquence complémentaire ;
- de 1 à 3 sondes, chevauchantes ou non, comprenant chacune tout ou une partie de la séquence SEQ ID NO : 35 ou de sa séquence complémentaire ;
- de 1 à 3 sondes, chevauchantes ou non, comprenant chacune tout ou une partie de la séquence SEQ ID NO : 230 ou de sa séquence complémentaire ;
- de 1 à 3 sondes, chevauchantes ou non, comprenant chacune tout ou une partie de la séquence SEQ ID NO : 316 ou de sa séquence complémentaire ;
- de 1 à 3 sondes, chevauchantes ou non, comprenant chacune tout ou une partie de la séquence SEQ ID NO : 341 ou de sa séquence complémentaire ;
- de 1 à 3 sondes, chevauchantes ou non, comprenant chacune tout ou une partie de la séquence SEQ ID NO : 454 ou de sa séquence complémentaire ;
- de 1 à 3 sondes, chevauchantes ou non, comprenant chacune tout ou une partie de la séquence SEQ ID NO : 593 ou de sa séquence complémentaire ;
- de 1 à 3 sondes, chevauchantes ou non, comprenant chacune tout ou une partie de la séquence SEQ ID NO : 664 ou de sa séquence complémentaire ;
- de 1 à 3 sondes, chevauchantes ou non, comprenant chacune tout ou une partie de la séquence SEQ ID NO : 666 ou de sa séquence complémentaire ;
- de 1 à 3 sondes, chevauchantes ou non, comprenant chacune tout ou une partie de la séquence SEQ ID NO : 811 ou de sa séquence complémentaire ;
- de 1 à 3 sondes, chevauchantes ou non, comprenant chacune tout ou une partie de la séquence SEQ ID NO : 855 ou de sa séquence complémentaire ;
- de 1 à 3 sondes, chevauchantes ou non, comprenant chacune tout ou une partie de la séquence SEQ ID NO : 951 ou de sa séquence complémentaire ;
- de 1 à 3 sondes, chevauchantes ou non, comprenant chacune tout ou une partie de la séquence SEQ ID NO : 1127 ou de sa séquence complémentaire ;
- de 1 à 3 sondes, chevauchantes ou non, comprenant chacune tout ou une partie de la séquence SEQ ID NO : 1136 ou de sa séquence complémentaire ;
- de 1 à 3 sondes, chevauchantes ou non, comprenant chacune tout ou une partie de la séquence SEQ ID NO : 1330 ou de sa séquence complémentaire ;
- de 1 à 3 sondes, chevauchantes ou non, comprenant chacune tout ou une partie de la séquence SEQ ID NO : 1498 ou de sa séquence complémentaire ; et
- de 1 à 3 sondes, chevauchantes ou non, comprenant chacune tout ou une partie de la séquence SEQ ID NO : 1752 ou de sa séquence complémentaire.

### Exemple 5. Protocole de détection

Les étapes du test diagnostique sont les suivantes:
L'échantillon est préparé par une étape d'extraction de l'ARN à partir d'échantillons de sang collectés dans des tubes de prélèvement sanguin Paxgene. Ces tubes contiennent un additif qui stabilise le profil de transcription de gènes in vivo en réduisant la dégradation d'ARN in vitro et en minimisant l'induction de gènes. Lorsqu'ils sont utilisés avec PaxgeneTM Blood RNA Kit, les échantillons prélevés permettent la détection et la quantification exactes du niveau de transcription de gènes. Après extraction, une étape de contrôle qualitatif et quantitatif de l'ARN est réalisée.
L'ARN est retro-transcrit, amplifié linéairement puis fragmenté, marqué à la biotine et enfin hybridé sur des Biopuces regroupant les sondes de l'invention. Les lames sont ensuite lavées, l'hybridation révélée puis et scannées pour mesurer le niveau d'hybridation sur chaque sonde. Les résultats d'hybridations sont importés et analysés dans le logiciel d'analyse statistique partek.
L'application de la signature donne une réponse binaire qui permet de placer le patient testé dans la catégorie AD ou dans la catégorie contrôle.

## Revendications

1. Méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins une sonde comprenant tout ou partie de chacune des séquences nucléiques cibles suivantes, ou de leur brin complémentaire : SEQ ID NOs : 34, 230, 341, 454, 664, 811, 951, 1127, 1136 et 1752, pour obtenir un profil d'hybridation, le profil d'hybridation étant caractéristique de la présence de la maladie d'Alzheimer chez ce mammifère.

2. Méthode selon la revendication 1, **caractérisée en ce que** l'ensemble de sondes comprend au moins une sonde comprenant tout ou partie de chacune des séquences nucléiques cibles suivantes, ou de leur brin complémentaire : SEQ ID NOs : 34, 35 ; 230, 316 ; 341, 454, 593 ; 664, 666 ; 811, 855 ; 951, 1127, 1136; 1330 ; 1498 et 1752.

3. Méthode selon la revendication 1 ou 2, **caractérisée en ce que** l'ensemble de sondes comprend au moins une sonde spécifique de chaque acide nucléique de l'un des panels 1 à 14 définis dans le tableau 2, ou d'un fragment distinctif de ceux-ci ayant au moins 15, de préférence au moins 16, 17, 18, 19, 20, 25 ou 30 bases consécutives, et/ou de leur brin complémentaire.

4. Méthode selon l'une quelconque des revendications précédentes, **caractérisée en ce que** l'ensemble de sondes comprend, pour chaque acide nucléique cible, un groupe de 1 à 3 sondes spécifiques partiellement chevauchantes ou non.

5. Méthode selon l'une quelconque des revendications précédentes, **caractérisée en ce que** les sondes sont immobilisées sur un support.

6. Méthode selon l'une quelconque des revendications précédentes, **caractérisée en ce que** le profil d'hybrisation est analysé par des méthodes informatiques.

7. Méthode selon l'une quelconque des revendications précédentes, **caractérisée en ce que** l'échantillon est un échantillon de sang total.

8. Produit comprenant un support sur lequel est immobilisé un ensemble de sondes, l'ensemble contenant au moins une sonde comprenant tout ou une partie de chacune des séquences nucléiques suivantes, ou de leur brin complémentaire : SEQ ID NOs : 34, 230, 341, 454, 664, 811, 951, 1127, 1136 et 1752.

9. Produit comprenant un support sur lequel sont immobilisées au moins deux sondes d'acide nucléique distinctes, chaque sonde comprenant une séquence complémentaire et/ou spécifique d'un acide nucléique cible choisi parmi SEQ ID NO: 1-1754 ou leur brin complémentaire.

10. Produit selon la revendication 9, comprenant un support sur lequel sont immobilisées au moins un ensemble de sondes d'acide nucléique distinctes comprenant une séquence complémentaire et/ou spécifique des acides nucléiques de l'un des panels 1 à 14 définis dans le tableau 2.

11. Produit selon la revendication 9, comprenant un support sur lequel est immobilisé un ensemble de sondes comprenant au moins une sonde d'acide nucléique complémentaire et/ou spécifique de chacun des acide nucléique de SEQ ID NO: 1-1754 ou de leur brin complémentaire.

12. Produit selon l'une des revendications 9 à 11, **caractérisé en ce que** les sondes sont choisies parmi SEQ ID : 1755-6532.

13. Kit comprenant un compartiment ou conteneur comprenant au moins 5, 10, 20, 30, 40, 50, 60 ou plus acides nucléiques différents choisis parmi les acides nucléiques définis dans l'une des revendications 1 à 12.

14. Kit comprenant un produit selon l'une des revendications 8 à 12 et des réactifs pour une réaction d'hybridation.

15. Utilisation d'un produit ou kit selon l'une des revendications 8 à 14 pour la détection in vitro ou ex vivo de la présence de la maladie d'Alzheimer, ou de la réponse à un traitement de la maladie d'Alzheimer chez un sujet.

16. Utilisation d'un ensemble de sondes tel que défini dans l'une des revendications 1 à 5 pour détecter *in vitro* ou *ex vivo* la présence de la maladie d'Alzheimer chez un sujet.

17. Méthode pour détecter la présence de la maladie d'Alzheimer chez un mammifère, comprenant la mise en contact, dans des conditions permettant une réaction d'amplification, des acides nucléiques issus d'un échantillon de sang du mammifère et d'un ensemble d'amorces, l'ensemble d'amorces comprenant au moins une amorce comprenant tout ou partie de chacune des séquences nucléiques cibles suivantes, ou de leur brin complémentaire : SEQ ID NOs : 34, 230, 341, 454, 664, 811, 951, 1127, 1136 et 1752, pour obtenir un profil ou produit d'amplification, le profil ou produit d'amplification étant caractéristique de la présence de la maladie d'Alzheimer chez ce mammifère.

18. Utilisation d'un ensemble d'amorces comprenant au moins une amorce comprenant tout ou partie de chacune des séquences nucléiques cibles mentionnées dans la revendication 17, pour la détection *in vitro* ou *ex vivo* de la maladie d'Alzheimer.
